# EUROPEAN PATENT SPECIFICATION

(11) **EP 0 355 978 B1**
(45) Date of publication and mention of the grant of the patent: **09.06.1993**
(21) Application number: 89307161.3
(22) Date of filing: 14.07.1989
(51) Int. Cl.: C07C 43/12, C07C 41/22, C07C 59/135

(54) **Process for preparing CHF2OCHFCF3 and CHF2OCHCLCF3 and novel intermediate compounds employed thereby**
Verfahren zur Herstellung von CHF20CHFCF3 und CHF20CHCLCF3 und dabei verwendete Zwischenverbindungen
Procédé pour la préparation de CHF20CHFCF3 et CHF20CHCLCF3 et composés intermédiaires utilisés à cet effet

(30) Priority: 18.07.1988 US 220129
(43) Date of publication of application: 28.02.1990
(73) Proprietor: Anaquest, Inc., Liberty Corner, NJ 07938-0804 (US)
(72) Inventor: Robin, Mark L., South Plainfield New Jersey 07080 (US); Halpern, Donald F., Fanwood New Jersey 07023 (US)
(74) Representative: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) References cited:
- EP-A- 0 285 237
- US-A- 3 535 388
- CHEMICAL ABSTRACTS, vol. 98, no. 15, 11th April 1983, page 626, abstract no. 125927n, Columbus, Ohio, US; H. MUFFLER et al.: "Cyclization in the presence of fluoride ions. 2. 4,5-Perfluoro-1,3-dioxolanes"

## Description

The present invention relates to the preparation of isoflurane and 2-(difluoromethoxy)-1,1,1,2-tetra-fluoroethane, which are inhalation anaesthetics that have received much attention because they provide a rapid rate of recovery and therefore are particularly suitable for administration to patients during outpatient surgery.

The most common method of preparing isoflurane (CHF₂OCHClCF₃) is by the reaction of trifluoroethanol (CF₃CH₂OH) and chlorodifluoromethane (CF₂HCl) in the presence of an organic base to produce a compound of the formula CHF₂OCH₂CF₃ which is then reacted with chlorine gas in the presence of light energy and optionally an organic solvent to thereby obtain isoflurane.

2-(difluoromethoxy)-1,1,1,2-tetrafluoroethane (hereinafter referred to as "CHF₂OCHFCF₃") is most commonly produced by reacting isoflurane with a fluorinating agent such as BrF₃. See Example 3 of EP-A-285 237. The production of CHF₂OCHFCF₃ is largely dependent on the starting materials used to prepare isoflurane.

The starting material, trifluoroethanol, is both difficult to produce and expensive to obtain. Use of this starting material therefore significantly increases the cost of producing both isoflurane and CHF₂OCHFCF₃. The invention provides new synthetic routes to isoflurane and 2-(difluoromethoxy)-1,1,1,2-tetra-fluoroethane via respectively the novel intermediate compounds CHC1₂OCH₂COCl and CHCl₂OCHClCOCl, which are coproducts of a reaction between sulphur tetrafluoride and methoxy acetyl chloride.

One process according to the present invention comprises reacting the novel compound CHCl₂OCHClCOCl with SF₄ at an elevated temperature and separating the product CHF₂OCHFCF₃. The reaction is conducted at elevated temperatures, preferably in the range of about 145 to 155°C. Isoflurane is also produced in this method and can be converted by known methods into CHF₂OCHFCF₃ or retained for use as an anaesthetic.

CHCl₂OCHClCOCl may be prepared by reacting CH₃OCH₂COOH with a chlorinating agent such as SOC1₂ or PC1₅ to produce the corresponding acid chloride which in turn is reacted with chlorine gas in the presence of light energy either neat or in an organic solvent such as carbon tetrachloride, to produce CHCl₂OCH₂COCl as a first novel intermediate compound and CHCl₂OCHClCOCl as a second novel intermediate compound.

The yield of the first and second novel intermediates is temperature dependent. In general the reaction may be conducted at a temperature in the range of about -15°C to the boiling point of the solvent, or in the absence of a solvent, to about 50°C.

An increased yield of the first novel intermediate is favoured by lower temperatures in the above described temperature range, preferably about 0° to 15°C, most preferably about 10°C.

On the other hand, for the direct production of CHF₂OCHFCF₃, increasing the yield of the second intermediate is desirable. accordingly, the reaction of CH₃OCH₂COCl and chlorine gas in the presence of light energy is conducted at higher temperatures than 10°C, preferably at least 20°C.

The second intermediate compound may be reacted with SF₄ at elevated temperatures in accordance with the invention to yield a mixture of the desired compound CHF₂OCHFCF₃ and isoflurane.

The invention also provides a process for preparing isoflurane by reacting a compound of the formula CHCl₂OCH₂COCl with sulphur tetrafluoride at an elevated temperature and then photochemically chlorinating the resulting compound to form isoflurane. This latter reaction may optionally be conducted in the presence of an organic solvent. The isoflurane thus produced may be converted to CHF₂OCHFCF₃ by reaction with a fluorinating agent such as bromine trifluoride.

CHF₂OCHFCF₃ is normally a clear, colourless, liquid having the following physical properties: boiling point 23.5°C, molecular weight 168, estimated vapour pressure 660 mmHG at 20°C, and a specific gravity of 1.44. IR shows a prominent peak at 4903cm⁻¹ and the ¹H NMR shows a triplet at 6.5ppm (J = 70Hz) and a doublet of quartets at 5.9ppm (J_{gem} = 56Hz, J_{vic} = 3Hz). The compound is non-flammable, and stable to soda lime, rendering it particularly suitable as an inhalation anaesthetic. Other characteristics and descriptions of CHF₂OCHFCF₃ and anaesthetic compositions containing the same as disclosed in US Patent No. 4762856, incorporated herein by reference.

The invention is now illustrated by the following example:

### EXAMPLE 1

### Production of CHCl₂OCH₂COCl From Methoxy Acetyl Chloride

18.9g of methoxy acetyl chloride (CH₃OCH₂COCl) and 200ml of CC1₄ were added to a 300ml reactor fitted with an outer cooling jacket. The temperature of the reactor was maintained at -5 to 5°C. Chlorine gas was gradually added to the reactor through a gas dispersion tube at the rate of 0.002 litre/min (0.057mg/min).

After approximately 16 hours, the chlorine gas flow was reduced to its lowest visual setting as evidenced by the presence of bubbles in the reaction solution and continued for 8 additional hours. The reaction mass was then warmed to room temperature and the CCl₄ distilled off at ambient pressure. The resulting product (37.6g) was transferred to a 50ml flask and vacuum distilled to produce a fraction having the following characteristics:
bp₅₂ₘₘ = to 89°C (10.4g)
The ¹NMR showed a 7.4 ppm singlet for CHCl₂-O- and a 4.6 ppm singlet for -O-CH₂COCL.

### EXAMPLE 2

### Production of CHCl₂OCHC1COCl From Methoxy Acetyl Chloride

20g of methoxy acetyl chloride and 168g of CCl₄ were added to the same reactor described in Example 1. The temperature of the reactor was maintained at about 30°C. Chlorine gas was gradually added to the reactor through a gas dispersion tube at approximately the same rate as described in Example 1 over approximately three hours.

The reaction mass was then allowed to warm to room temperature and the CCl₄ was distilled off at ambient pressure. The resulting product was vacuum distilled to produce a fraction having the following characteristics:
Bp_{(2 to 4.7mm)} = 42-55°C (17.2g)

The ¹H NMR showed a 7.7 ppm singlet for CHCl₂-O- and a 5.9 ppm singlet for -OCHC1COCl.

### EXAMPLE 3

### Reaction of CHCl₂OCHClCOCl with SF₄

A stainless steel tube reactor containing 6.3g (0.03 moles) of CHCl₂OCHClCOCl produced in Example 1 was cooled with liquid nitrogen and 13.3g (0.06 moles) of SF₄ was condensed into the stainless steel tube reactor. The reactor was warmed to room temperature and then heated to and maintained at a temperature of 145-155°C for 6 hours during which time the resulting pressure was from 600-650 psig.

The reactor was then cooled to room temperature and the resulting gaseous products were collected in a scrubber containing a 9% NaOH solution maintained at 10°C. The reactor was then heated to 80°C and additional distillate collected in the NaOH scrubber. The scrubber was then heated to 100°C to yield an organic phase which was collected in a Dean-Stark trap cooled to 0°C. Two cuts of 2.9g and 0.5g were collected in the trap. The cuts were analysed by gas chromatography and found to have the following composition:

| Component | Wt(g) | Moles | %Yield |
|---|---|---|---|
| CHF₂OCHFCF₃ | 1.44 | 0.0086 | 29% |
| isoflurane | 1.47 | 0.0080 | 27% |

### EXAMPLE 4

### Reaction of CHCl₂OCH₂COCl with SF₄ and conversion of the product to isoflurane

A stainless steel tube reactor containing 10.4g (0.059 moles) of CHCl₂OCH₂COCl produced in Example 2 was cooled with liquid nitrogen and 14.6g (0.07 moles) of SF₄ was condensed into the tube reactor. The reactor was allowed to warm to room temperature and then gradually warmed to 132°C over the course of 2½ hours. The pressure in the reactor increased from 95 to 505 psig.

Thereafter the temperature of the reactor was gradually raised to between 143 to 163°C for about 1 hour causing the pressure in the reactor to increase to between 585 to 645 psi. The reactor was then maintained at a temperature between 145 to 155°C for about 4 hours at an autogenous pressure of between 595 to 605 psig.

The reactor was then cooled to room temperature and the resulting gases were vented into a scrubber containing a solution of 50g to 50% NaOH in 320g of water at a temperature of -10°C.

The resulting organic layer (8.7g) was separated from the aqueous phase. Gas chromatography showed a retention time (3.97min) similar to that of CF₃CH₂OCHF₂. The product was separately tested to that of CF₃CH₂OCHF₂. The product was separately tested in a mass spectrophotometer and ¹H NMR and the above structure was confirmed. The yield of the product was 69%. The resulting product can be readily converted to isoflurane (CHF₂OCHClCF₃) by reaction with chlorine gas in the presence of light energy.

The product (CF₃CH₂OCHF₂,129g) was placed into a small chorination apparatus equipped with a "Dry-Ice" trap which had been purged with nitrogen for two minutes. Gaseous chlorine was bubbled through the liquid at 15°C while it was irradiated with incandescant light. The effluent HC1 produced was titrated until approximately one mole was collected. The reaction product, which weighed 140g, was distilled through a 60x2 cm stainless steel packed column to yield pure CF₃CHC1OCHF₂, bp 48°-48.5°C at 760mm. The structure was determined by nmr and elemental analysis.

It is not essential to separate a mixture of CHC1₂OCHC1COCL and CHC1₂OCH₂COC1 before conducting a reaction with sulphur tetrafluoride. The reaction of sulphur tetrafluoride with the unseparated mixture may be performed under the conditions set out in Example 3 or Example 4. The resulting mixture of CHF₂OCHFCF₃, CHF₂OCH₂CF₃ and CHF₂OCHClCF₃ may then be separated into its components by fractional distillation. CHF₂OCH₂CF₃ may be photochemically converted to form additional isoflurane by the procedure set out in the immediately preceding paragraph.

## Claims

1. A process for preparing a compound of the formula CHF₂OCHFCF₃ comprising reacting a compound having the formula CHCl₂OCHClCOCl with sulphur tetrafluoride at an elevated temperature and separating the product CHF₂OCHFCF₃.

2. A process according to claim 1, wherein the product is separated by fractional distillation.

3. A process for preparing isoflurane by reacting a compound of the formula CHCl₂OCH₂COCl with sulphur tetrafluoride at an elevated temperature to form CHF₂OCH₂CF₃ and then photochemically chlorinating the resulting compound to form isoflurane.

4. A process according to claim 3, in which the isoflurane is converted to CHF₂OCHFCF₃ by reaction with a fluorinating agent.

5. A process as claimed in any one of claims 1 to 4 in which the reaction with sulphur tetrafluoride is conducted at a temperature in the range 145 to 155°C.

6. A process according to any one of the preceding claims in which compounds having the formulae CHCl₂OCHClCOCl and CHCl₂OCH₂COCl are prepared by reacting CH₃OCH₂COCl with chlorine in the presence of light energy and in the presence or absence of a solvent at a temperature in the range of -15°C up to the boiling point of the solvent or, if no solvent is present up to about 50°C, and then separating the resulting mixture to obtain CHCl₂OCHClCOCl or CHCl₂OCH₂COCl.

7. A process according to claim 6 in which the mixture is separated by distillation.

8. A process according to claim 6 or claim 7, in which the reaction between CH₃OCH₂COCl and chlorine is conducted at a temperature of from 0 to 15°C to favour the formation of CHCl₂OCH₂COCl.

9. A process according to claim 8, wherein the reaction between CH₃OCH₂COCl and chlorine is conducted at a temperature of at least 20°C thereby to favour the formation of CHCl₂OCHClCOCl.

10. A compound of the formula CHCl₂OCH₂COCl.

11. A compound of the formula CHCl₂OCHClCOCl.

12. A process comprising reacting CH₃OCH₂COC1 with chlorine in the presence of light energy and in the presence or absence of a solvent at a temperature in the range of from -15°C up to the boiling point of the solvent, or if no solvent is present, up to about 50°C to form a mixture of CHC1₂OCHC1COC1 and CHC1₂OCH₂COC1; reacting said mixture with sulphur tetrafluoride at an elevated temperature to form a mixture of CHF₂OCHFCF₃, CHF₂OCH₂CF₃ and isoflurane; and separating said mixture by distillation.

13. A process in accordance with Claim 12, wherein said recovered CHF₂OCH₂CF₃ is photochemically chlorinated to form additional isoflurane.

14. A process in accordance with Claim 13, wherein the isoflurane is converted to CHF₂OCHFCF₃ by reaction with a fluorinating agent.

## Patentansprüche

1. Verfahren zur Herstellung einer Verbindung der Formel CHF₂OCHFCF₃, bei dem eine Verbindung der Formel CHCl₂OCHClCOCl mit Schwefeltetrafluorid bei einer erhöhten Temperatur umgesetzt und das Produkt CHF₂OCHFCF₃ abgetrennt wird.

2. Verfahren nach Anspruch 1, bei dem das Produkt durch fraktionierte Destillation abgetrennt wird.

3. Verfahren zur Herstellung von Isofluran, bei dem eine Verbindung der Formel CHCl₂OCH₂COCl mit Schwefeltetrafluorid bei einer erhöhten Temperatur unter Bildung von CHF₂OCH₂CF₃ umgesetzt wird und dann die entstandene Verbindung photochemisch chloriert wird unter Bildung von Isofluran.

4. Verfahren nach Anspruch 3, bei dem das Isofluran durch Umsetzung mit einem Fluorierungsmittel in CHF₂OCHFCF₃ umgewandelt wird.

5. Verfahren nach einem der Ansprüche 1 bis 4, bei dem die Umsetzung mit Schwefeltetrafluorid bei einer Temperatur im Bereich von 145 bis 155°C durchgeführt wird.

6. Verfahren nach einem der vorausgehenden Ansprüche, bei dem Verbindungen der Formeln CHCl₂OCHClCOCl und CHCl₂OCH₂COCl durch Umsetzung von CH₃OCH₂COCl mit Chlor in Anwesenheit von Lichtenergie und in Anwesenheit oder Abwesenheit eines Lösemittels bei einer Temperatur im Bereich von -15°C bis zum Siedepunkt des Lösemittels oder, falls kein Lösemittel anwesend ist, bis etwa 50°C hergestellt werden und dann die entstandene Mischung getrennt wird, um CHCl₂OCHClCOCl oder CHCl₂OCH₂COCl zu erhalten.

7. Verfahren nach Anspruch 6, bei dem die Mischung durch Destillation getrennt wird.

8. Verfahren nach Anspruch 6 oder Anspruch 7, bei dem die Umsetzung von CH₃OCH₂COCl mit Chlor bei einer Temperatur von 0 bis 15°C durchgeführt wird, um die Bildung von CHCl₂OCH₂COCl zu begünstigen.

9. Verfahren nach Anspruch 8, bei dem die Umsetzung von CH₃OCH₂COCl mit Chlor bei einer Temperatur von wenigstens 20°C durchgeführt wird, um dadurch die Bildung von CHCl₂OCHClCOCl zu begünstigen.

10. Verbindung der Formel CHCl₂OCH₂COCl.

11. Verbindung der Formel CHCl₂OCHClCOCl.

12. Verfahren, bei dem CH₃OCH₂COCl mit Chlor in Anwesenheit von Lichtenergie und in Anwesenheit oder Abwesenheit eines Lösemittels bei einer Temperatur im Bereich von -15°C bis zum Siedepunkt des Lösemittels oder, wenn kein Lösemittel anwesend ist, bis etwa 50°C unter Bildung einer Mischung von CHCl₂OCHClCOCl und CHCl₂OCH₂COCl umgesetzt wird, diese Mischung mit Schwefeltetrafluorid bei einer erhöhten Temperatur umgesetzt wird unter Bildung einer Mischung von CHF₂OCHFCF₃, CHF₂OCH₂CF₃ und Isofluran und diese Mischung durch Destillation getrennt wird.

13. Verfahren nach Anspruch 12, bei dem das gewonnene CHF₂OCH₂CF₃ photochemisch chloriert wird unter Bildung von zusätzlichem Isofluran.

14. Verfahren nach Anspruch 13, bei dem das Isofluran durch Umsetzung mit einem Fluorierungsmittel in CHF₂OCHFCF₃ umgewandelt wird.

## Revendications

1. Procédé pour préparer un composé ayant la formule CHF₂OCHFCF₃ comprenant les étapes consistant à faire réagir un composé ayant la formule CHCl₂OCHClCOCl avec du tétrafluorure de soufre à une température élevée et à séparer le produit CHF₂OCHFCF₃.

2. Procédé selon la revendication 1, dans lequel le produit est séparé par distillation fractionnée.

3. Procédé pour la préparation d'isoflurane comprenant les étapes consistant à faire réagir un composé ayant la formule CHCl₂OCH₂COCl avec du tétrafluorure de soufre à une température élevée pour former du CHF₂OCH₂CF₃, et à chlorer ensuite par voie photochimique le composé résultant afin de former de l'isoflurane.

4. Procédé selon la revendication 3, dans lequel l'isoflurane est converti en CHF₂OCHFCF₃ par réaction avec un agent de fluorination.

5. Procédé selon l'une quelconque des revendications 1 à 4, dans lequel la réaction avec le tétrafluorure de soufre est menée à une température dans la plage de 145 à 155°C.

6. Procédé selon l'une quelconque des revendications précédentes, dans lequel on prépare des composés ayant les formules CHCl₂OCHClCOCl et CHCl₂OCH₂COCl en faisant réagir du CH₃OCH₂COCl avec du chlore en présence d'énergie lumineuse et en présence ou absence d'un solvant à une température dans la plage de -15°C jusqu'au point d'ébullition du solvant ou, jusqu'à environ 50°C si aucun solvant n'est présent, et on sépare ensuite le mélange résultant pour obtenir du CHCl₂OCHClCOCl ou du CHCl₂OCH₂COCl.

7. Procédé selon la revendication 6, dans lequel le mélange est séparé par distillation.

8. Procédé selon l'une ou l'autre des revendications 6 ou 7, dans lequel on conduit la réaction entre le CH₃OCH₂COCl et le chlore à une température comprise entre 0 et 15°C pour favoriser la formation de CHCl₂OCH₂COCl.

9. Procédé selon la revendication 8, dans lequel on conduit la réaction entre le CH₃OCH₂COCl et le chlore à une température d'au moins 20°C pour favoriser ainsi la formation de CHCl₂OCHClCOCl.

10. Composé ayant la formule CHCl₂OCH₂COCl.

11. Composé ayant la formule CHCl₂OCHClCOCl.

12. Procédé comprenant les étapes consistant à faire réagir du CH₃OCH₂COCl avec du chlore en présence d'énergie lumineuse et en présence ou absence d'un solvant à une température dans la plage de -15°C jusqu'au point d'ébullition du solvant, ou jusqu'à environ 50°C si aucun solvant n'est présent, pour former un mélange de CHCl₂OCHClCOCl et de CHCl₂OCH₂COCl; à faire réagir ledit mélange avec du tétrafluorure de soufre à une température élevée pour former un mélange de CHF₂OCHFCF₃, CHF₂OCH₂CF₃ et d'isoflurane; et à séparer ledit mélange par distillation.

13. Procédé selon la revendication 12, dans lequel ledit CHF₂OCH₂CF₃ obtenu est chloré par voie photochimique pour former de l'isoflurane additionnel.

14. Procédé selon la revendication 13, dans lequel l'isoflurane est converti en CHF₂OCHFCF₃ par réaction avec un agent de fluorination.
